# EUROPEAN PATENT APPLICATION

(11) **EP 1 586 644 A2**
(43) Date of publication of application: **19.10.2005**
(21) Application number: 05012435.3
(22) Date of filing: 09.08.2000
(51) Int. Cl.: C12N 15/12, A61K 38/17, A61K 31/40, A61K 31/70, G01N 33/50, A61P 9/00, A61P 35/00

(54) **Medicament for the prevention and/or the treatment of ischemic heart and peripheral vascular diseases, tumour development and for wound healing**

(30) Priority: 09.08.1999 EP 99870171
(62) Divisional of application: 00951488.6
(71) Applicant: UNIVERSITE CATHOLIQUE DE LOUVAIN, 1348 Louvain la Neuve (BE)
(72) Inventor: Balligand, Jean-Luc, 1950 Kraainem (BE); Feron, Olivier, 1970 Wezembeck-Oppem (BE)
(74) Representative: Brants, Johan P.E.

(57) **Abstract**

The present invention is related to a compound or a pharmaceutical composition thereof for the use of a medicament in the modulation of angiogenesis through the tackling of the intracellular free cholesterol-caveolin1-eNOS-NO pathway. The present invention also provides a method of study, testing, screening and manufacturing of new compounds or compositions which influences the angiogenesis via said pathway with the aim of identifying a compound or composition having possible therapeutic or prophylactic properties upon ischemic heart and peripheral vascular diseases including cerebral diseases, tumour development and wound healing.

SEQUENCE LISTING

<110> Université Catholique de Louvain

<120> Use of compound or pharmaceutical composition for the
      prevention and/or the treatment of ischemic heart and
      peripheral vascular diseases, tumour development and
      for wound healing

<130> UCL-012-EP-DIV1

<140> PCT/EP00/07731
<141> 2000-08-09

<150> 99870171.8
<151> 1999-08-09

<160> 86

<170> PatentIn Ver. 2.1

<210> 1
<211> 537
<212> DNA
<213> Homo sapiens

<400> 1
atgtctgggg gcaaatacgt agactcggag ggacatctct acaccgttcc catccgggaa 60
cagggcaaca tctacaagcc caacaacaag gccatggcag acgagctgag cgagaagcaa 120
gtgtacgacg cgcacaccaa ggagatcgac ctggtcaacc gcgaccctaa acacctcaac 180
gatgacgtgg tcaagattga ctttgaagat gtgattgcag aaccagaagg gacacacagt 240
tttcacggca tttggaaggc cagcttcacc accttcactg tgacgaaata ctggttttac 300
cgcttgctgt ctgccctctt tggcatcccg atggcactca tctggggcat ttacttcgcc 360
attctctctt tcctgcacat ctgggcagtt gtaccatgca ttaagagctt cctgattgag 420
attcagtgca ccagccgtgt ctattccatc tacgtccaca ccgtctgtga cccactcttt 480
gaagctgttg ggaaaatatt cagcaatgtc cgcatcaact tgcagaaaga aatataa    537

<210> 2
<211> 20
<212> PRT
<213> Homo sapiens

<400> 2
His Gly Ile Trp Lys Ala Ser Phe Thr Thr Phe Thr Val Thr Lys Tyr
  1               5                  10                  15

Trp Phe Tyr Arg
             20

<210> 3
<211> 22
<212> PRT
<213> Homo sapiens

<400> 3
Lys Ser Phe Leu Ile Glu Ile Gln Cys Thr Ser Arg Val Tyr Ser Ile
  1               5                  10                  15

Tyr Val His Thr Val Cys
             20

<210> 4
<211> 10
<212> PRT
<213> Artificial Sequence

<220>
<223> Description of Artificial Sequence:caveolin
      binding motif

<400> 4

Phe Pro Ala Ala Pro Phe Ser Gly Trp Tyr
1               5                   10

<210> 5
<211> 40
<212> DNA
<213> Artificial Sequence

<220>
<223> Description of Artificial Sequence:partial
      antisense sequence of human caveolin-1

<400> 5
gagtctacgt atttgccccc agacatgctg gcccgtggct                              40

<210> 6
<211> 10
<212> PRT
<213> Artificial Sequence

<220>
<223> Description of Artificial Sequence:caveolin
      binding motif

<400> 6
Phe Xaa Phe Xaa Xaa Xaa Xaa Phe Xaa Phe
1               5                   10

<210> 7
<211> 10
<212> PRT
<213> Artificial Sequence

<220>
<223> Description of Artificial Sequence:caveolin
      binding motif

<400> 7
Phe Xaa Phe Xaa Xaa Xaa Xaa Phe Xaa Tyr
1               5                   10

<210> 8
<211> 10
<212> PRT
<213> Artificial Sequence

<220>
<223> Description of Artificial Sequence:caveolin
      binding motif

<400> 8
Phe Xaa Phe Xaa Xaa Xaa Xaa Tyr Xaa Tyr
1               5                   10

<210> 9
<211> 10
<212> PRT
<213> Artificial Sequence

<220>
<223> Description of Artificial Sequence:caveolin
      binding motif

<400> 9
Phe Xaa Tyr Xaa Xaa Xaa Xaa Tyr Xaa Tyr
1               5                   10

<210> 10
<211> 10
<212> PRT
<213> Artificial Sequence

<220>
<223> Description of Artificial Sequence:caveolin
      binding motif

<400> 10
Phe Xaa Trp Xaa Xaa Xaa Xaa Tyr Xaa Tyr
  1               5                   10

<210> 11
<211> 10
<212> PRT
<213> Artificial Sequence

<220>
<223> Description of Artificial Sequence:caveolin
      binding motif

<400> 11
Phe Xaa Phe Xaa Xaa Xaa Xaa Trp Xaa Tyr
  1               5                   10

<210> 12
<211> 10
<212> PRT
<213> Artificial Sequence

<220>
<223> Description of Artificial Sequence:caveolin
      binding motif

<400> 12
Phe Xaa Tyr Xaa Xaa Xaa Xaa Trp Xaa Tyr
  1               5                   10

<210> 13
<211> 10
<212> PRT
<213> Artificial Sequence

<220>
<223> Description of Artificial Sequence:caveolin
      binding motif

<400> 13
Phe Xaa Tyr Xaa Xaa Xaa Xaa Trp Xaa Tyr
  1               5                   10

<210> 14
<211> 10
<212> PRT
<213> Artificial Sequence

<220>
<223> Description of Artificial Sequence:caveolin
      binding motif

<400> 14
Phe Xaa Tyr Xaa Xaa Xaa Xaa Phe Xaa Tyr
  1               5                   10

<210> 15
<211> 10
<212> PRT
<213> Artificial Sequence

<220>
<223> Description of Artificial Sequence:caveolin
      binding motif

<400> 15
Phe Xaa Trp Xaa Xaa Xaa Xaa Phe Xaa Tyr
  1               5                   10


<210> 16
<211> 10
<212> PRT
<213> Artificial Sequence

<220>
<223> Description of Artificial Sequence:caveolin
      binding motif

<400> 16
Phe Xaa Phe Xaa Xaa Xaa Xaa Phe Xaa Trp
  1               5                   10


<210> 17
<211> 10
<212> PRT
<213> Artificial Sequence

<220>
<223> Description of Artificial Sequence:caveolin
      binding motif

<400> 17
Phe Xaa Phe Xaa Xaa Xaa Xaa Tyr Xaa Trp
  1               5                   10


<210> 18
<211> 10
<212> PRT
<213> Artificial Sequence

<220>
<223> Description of Artificial Sequence:caveolin
      binding motif

<400> 18
Phe Xaa Tyr Xaa Xaa Xaa Xaa Tyr Xaa Trp
  1               5                   10


<210> 19
<211> 10
<212> PRT
<213> Artificial Sequence

<220>
<223> Description of Artificial Sequence:caveolin
      binding motif

<400> 19
Phe Xaa Trp Xaa Xaa Xaa Xaa Tyr Xaa Trp
  1               5                   10

<210> 20
<211> 10
<212> PRT
<213> Artificial Sequence

<220>
<223> Description of Artificial Sequence:caveolin
      binding motif

<400> 20
Phe Xaa Phe Xaa Xaa Xaa Xaa Trp Xaa Trp
  1               5                  10


<210> 21
<211> 10
<212> PRT
<213> Artificial Sequence

<220>
<223> Description of Artificial Sequence:caveolin
      binding motif

<400> 21
Phe Xaa Tyr Xaa Xaa Xaa Xaa Trp Xaa Trp
  1               5                  10


<210> 22
<211> 10
<212> PRT
<213> Artificial Sequence

<220>
<223> Description of Artificial Sequence:caveolin
      binding motif

<400> 22
Phe Xaa Trp Xaa Xaa Xaa Xaa Trp Xaa Trp
  1               5                  10


<210> 23
<211> 10
<212> PRT
<213> Artificial Sequence

<220>
<223> Description of Artificial Sequence:caveolin
      binding motif

<400> 23
Phe Xaa Tyr Xaa Xaa Xaa Xaa Phe Xaa Trp
  1               5                  10


<210> 24
<211> 10
<212> PRT
<213> Artificial Sequence

<220>
<223> Description of Artificial Sequence:caveolin
      binding motif

<400> 24
Phe Xaa Trp Xaa Xaa Xaa Xaa Phe Xaa Trp
  1               5                  10


<210> 25

<211> 10
<212> PRT
<213> Artificial Sequence

<220>
<223> Description of Artificial Sequence:caveolin
      binding motif

<400> 25
Phe Xaa Phe Xaa Xaa Xaa Xaa Tyr Xaa Phe
  1               5                   10


<210> 26
<211> 10
<212> PRT
<213> Artificial Sequence

<220>
<223> Description of Artificial Sequence:caveolin
      binding motif

<400> 26
Phe Xaa Tyr Xaa Xaa Xaa Xaa Tyr Xaa Phe
  1               5                   10


<210> 27
<211> 10
<212> PRT
<213> Artificial Sequence

<220>
<223> Description of Artificial Sequence:caveolin
      binding motif

<400> 27
Phe Xaa Trp Xaa Xaa Xaa Xaa Tyr Xaa Phe
  1               5                   10


<210> 28
<211> 10
<212> PRT
<213> Artificial Sequence

<220>
<223> Description of Artificial Sequence:caveolin
      binding motif

<400> 28
Phe Xaa Phe Xaa Xaa Xaa Xaa Trp Xaa Phe
  1               5                   10


<210> 29
<211> 10
<212> PRT
<213> Artificial Sequence

<220>
<223> Description of Artificial Sequence:caveolin
      binding motif

<400> 29
Phe Xaa Tyr Xaa Xaa Xaa Xaa Trp Xaa Phe
  1               5                   10


<210> 30
<211> 10

<212> PRT
<213> Artificial Sequence

<220>
<223> Description of Artificial Sequence:caveolin
      binding motif

<400> 30
Phe Xaa Trp Xaa Xaa Xaa Xaa Trp Xaa Phe
  1               5                   10


<210> 31
<211> 10
<212> PRT
<213> Artificial Sequence

<220>
<223> Description of Artificial Sequence:caveolin
      binding motif

<400> 31
Phe Xaa Tyr Xaa Xaa Xaa Xaa Phe Xaa Phe
  1               5                   10


<210> 32
<211> 10
<212> PRT
<213> Artificial Sequence

<220>
<223> Description of Artificial Sequence:caveolin
      binding motif

<400> 32
Phe Xaa Trp Xaa Xaa Xaa Xaa Phe Xaa Phe
  1               5                   10


<210> 33
<211> 10
<212> PRT
<213> Artificial Sequence

<220>
<223> Description of Artificial Sequence:caveolin
      binding motif

<400> 33
Tyr Xaa Phe Xaa Xaa Xaa Xaa Phe Xaa Phe
  1               5                   10


<210> 34
<211> 10
<212> PRT
<213> Artificial Sequence

<220>
<223> Description of Artificial Sequence:caveolin
      binding motif

<400> 34
Tyr Xaa Phe Xaa Xaa Xaa Xaa Phe Xaa Tyr
  1               5                   10


<210> 35
<211> 10
<212> PRT

<213> Artificial Sequence

<220>
<223> Description of Artificial Sequence:caveolin
      binding motif

<400> 35
Tyr Xaa Phe Xaa Xaa Xaa Xaa Tyr Xaa Tyr
  1               5                  10

<210> 36
<211> 10
<212> PRT
<213> Artificial Sequence

<220>
<223> Description of Artificial Sequence:caveolin
      binding motif

<400> 36
Tyr Xaa Tyr Xaa Xaa Xaa Xaa Tyr Xaa Tyr
  1               5                  10

<210> 37
<211> 10
<212> PRT
<213> Artificial Sequence

<220>
<223> Description of Artificial Sequence:caveolin
      binding motif

<400> 37
Tyr Xaa Trp Xaa Xaa Xaa Xaa Tyr Xaa Tyr
  1               5                  10

<210> 38
<211> 10
<212> PRT
<213> Artificial Sequence

<220>
<223> Description of Artificial Sequence:caveolin
      binding motif

<400> 38
Tyr Xaa Phe Xaa Xaa Xaa Xaa Trp Xaa Tyr
  1               5                  10

<210> 39
<211> 10
<212> PRT
<213> Artificial Sequence

<220>
<223> Description of Artificial Sequence:caveolin
      binding motif

<400> 39
Tyr Xaa Tyr Xaa Xaa Xaa Xaa Trp Xaa Tyr
  1               5                  10

<210> 40
<211> 10
<212> PRT
<213> Artificial Sequence

<220>
<223> Description of Artificial Sequence:caveolin
      binding motif

<400> 40
Tyr Xaa Trp Xaa Xaa Xaa Xaa Trp Xaa Tyr
  1               5                   10

<210> 41
<211> 10
<212> PRT
<213> Artificial Sequence

<220>
<223> Description of Artificial Sequence:caveolin
      binding motif

<400> 41
Tyr Xaa Phe Xaa Xaa Xaa Xaa Phe Xaa Trp
  1               5                   10

<210> 42
<211> 10
<212> PRT
<213> Artificial Sequence

<220>
<223> Description of Artificial Sequence:caveolin
      binding motif

<400> 42
Tyr Xaa Phe Xaa Xaa Xaa Xaa Tyr Xaa Trp
  1               5                   10

<210> 43
<211> 10
<212> PRT
<213> Artificial Sequence

<220>
<223> Description of Artificial Sequence:caveolin
      binding motif

<400> 43
Tyr Xaa Tyr Xaa Xaa Xaa Xaa Tyr Xaa Trp
  1               5                   10

<210> 44
<211> 10
<212> PRT
<213> Artificial Sequence

<220>
<223> Description of Artificial Sequence:caveolin
      binding motif

<400> 44
Tyr Xaa Trp Xaa Xaa Xaa Xaa Tyr Xaa Trp
  1               5                   10

<210> 45
<211> 10
<212> PRT
<213> Artificial Sequence

<220>
<223> Description of Artificial Sequence:caveolin
      binding motif

<400> 45
Tyr Xaa Phe Xaa Xaa Xaa Xaa Trp Xaa Trp
  1               5                   10


<210> 46
<211> 10
<212> PRT
<213> Artificial Sequence

<220>
<223> Description of Artificial Sequence:caveolin
      binding motif

<400> 46
Tyr Xaa Tyr Xaa Xaa Xaa Xaa Trp Xaa Trp
  1               5                   10


<210> 47
<211> 10
<212> PRT
<213> Artificial Sequence

<220>
<223> Description of Artificial Sequence:caveolin
      binding motif

<400> 47
Tyr Xaa Trp Xaa Xaa Xaa Xaa Trp Xaa Trp
  1               5                   10


<210> 48
<211> 10
<212> PRT
<213> Artificial Sequence

<220>
<223> Description of Artificial Sequence:caveolin
      binding motif

<400> 48
Tyr Xaa Phe Xaa Xaa Xaa Xaa Tyr Xaa Phe
  1               5                   10


<210> 49
<211> 10
<212> PRT
<213> Artificial Sequence

<220>
<223> Description of Artificial Sequence:caveolin
      binding motif

<400> 49
Tyr Xaa Tyr Xaa Xaa Xaa Xaa Tyr Xaa Phe
  1               5                   10


<210> 50
<211> 10
<212> PRT
<213> Artificial Sequence

<220>

<223> Description of Artificial Sequence:caveolin
      binding motif

<400> 50
Tyr Xaa Trp Xaa Xaa Xaa Xaa Tyr Xaa Phe
  1               5                   10

<210> 51
<211> 10
<212> PRT
<213> Artificial Sequence

<220>
<223> Description of Artificial Sequence:caveolin
      binding motif

<400> 51
Tyr Xaa Phe Xaa Xaa Xaa Xaa Trp Xaa Phe
  1               5                   10

<210> 52
<211> 10
<212> PRT
<213> Artificial Sequence

<220>
<223> Description of Artificial Sequence:caveolin
      binding motif

<400> 52
Tyr Xaa Tyr Xaa Xaa Xaa Xaa Trp Xaa Phe
  1               5                   10

<210> 53
<211> 10
<212> PRT
<213> Artificial Sequence

<220>
<223> Description of Artificial Sequence:caveolin
      binding motif

<400> 53
Tyr Xaa Trp Xaa Xaa Xaa Xaa Trp Xaa Phe
  1               5                   10

<210> 54
<211> 10
<212> PRT
<213> Artificial Sequence

<220>
<223> Description of Artificial Sequence:caveolin
      binding motif

<400> 54
Tyr Xaa Tyr Xaa Xaa Xaa Xaa Phe Xaa Phe
  1               5                   10

<210> 55
<211> 10
<212> PRT
<213> Artificial Sequence

<220>
<223> Description of Artificial Sequence:caveolin

binding motif

<400> 55
Tyr Xaa Trp Xaa Xaa Xaa Xaa Phe Xaa Phe
1               5                   10

<210> 56
<211> 10
<212> PRT
<213> Artificial Sequence

<220>
<223> Description of Artificial Sequence:caveolin
      binding motif

<400> 56
Tyr Xaa Tyr Xaa Xaa Xaa Xaa Phe Xaa Tyr
1               5                   10

<210> 57
<211> 10
<212> PRT
<213> Artificial Sequence

<220>
<223> Description of Artificial Sequence:caveolin
      binding motif

<400> 57
Tyr Xaa Trp Xaa Xaa Xaa Xaa Phe Xaa Tyr
1               5                   10

<210> 58
<211> 10
<212> PRT
<213> Artificial Sequence

<220>
<223> Description of Artificial Sequence:caveolin
      binding motif

<400> 58
Tyr Xaa Tyr Xaa Xaa Xaa Xaa Phe Xaa Trp
1               5                   10

<210> 59
<211> 10
<212> PRT
<213> Artificial Sequence

<220>
<223> Description of Artificial Sequence:caveolin
      binding motif

<400> 59
Tyr Xaa Trp Xaa Xaa Xaa Xaa Phe Xaa Trp
1               5                   10

<210> 60
<211> 10
<212> PRT
<213> Artificial Sequence

<220>
<223> Description of Artificial Sequence:caveolin
      binding motif

<400> 60
Trp Xaa Phe Xaa Xaa Xaa Xaa Phe Xaa Phe
1               5                   10

<210> 61
<211> 10
<212> PRT
<213> Artificial Sequence

<220>
<223> Description of Artificial Sequence:caveolin
      binding motif

<400> 61
Trp Xaa Phe Xaa Xaa Xaa Xaa Phe Xaa Tyr
1               5                   10

<210> 62
<211> 10
<212> PRT
<213> Artificial Sequence

<220>
<223> Description of Artificial Sequence:caveolin
      binding motif

<400> 62
Trp Xaa Phe Xaa Xaa Xaa Xaa Tyr Xaa Tyr
1               5                   10

<210> 63
<211> 10
<212> PRT
<213> Artificial Sequence

<220>
<223> Description of Artificial Sequence:caveolin
      binding motif

<400> 63
Trp Xaa Tyr Xaa Xaa Xaa Xaa Tyr Xaa Tyr
1               5                   10

<210> 64
<211> 10
<212> PRT
<213> Artificial Sequence

<220>
<223> Description of Artificial Sequence:caveolin
      binding motif

<400> 64
Trp Xaa Trp Xaa Xaa Xaa Xaa Tyr Xaa Tyr
1               5                   10

<210> 65
<211> 10
<212> PRT
<213> Artificial Sequence

<220>
<223> Description of Artificial Sequence:caveolin
      binding motif

<400> 65
Trp Xaa Phe Xaa Xaa Xaa Xaa Trp Xaa Tyr
1               5                   10

<210> 66
<211> 10
<212> PRT
<213> Artificial Sequence

<220>
<223> Description of Artificial Sequence:caveolin
      binding motif

<400> 66
Trp Xaa Tyr Xaa Xaa Xaa Xaa Trp Xaa Tyr
1               5                   10

<210> 67
<211> 10
<212> PRT
<213> Artificial Sequence

<220>
<223> Description of Artificial Sequence:caveolin
      binding motif

<400> 67
Trp Xaa Trp Xaa Xaa Xaa Xaa Trp Xaa Tyr
1               5                   10

<210> 68
<211> 10
<212> PRT
<213> Artificial Sequence

<220>
<223> Description of Artificial Sequence:caveolin
      binding motif

<400> 68
Trp Xaa Phe Xaa Xaa Xaa Xaa Phe Xaa Trp
1               5                   10

<210> 69
<211> 10
<212> PRT
<213> Artificial Sequence

<220>
<223> Description of Artificial Sequence:caveolin
      binding motif

<400> 69
Trp Xaa Phe Xaa Xaa Xaa Xaa Tyr Xaa Trp
1               5                   10

<210> 70
<211> 10
<212> PRT
<213> Artificial Sequence

<220>
<223> Description of Artificial Sequence:caveolin
      binding motif

<400> 70

Trp Xaa Tyr Xaa Xaa Xaa Xaa Tyr Xaa Trp
1               5                   10

<210> 71
<211> 10
<212> PRT
<213> Artificial Sequence

<220>
<223> Description of Artificial Sequence:caveolin
      binding motif

<400> 71
Trp Xaa Trp Xaa Xaa Xaa Xaa Tyr Xaa Trp
1               5                   10

<210> 72
<211> 10
<212> PRT
<213> Artificial Sequence

<220>
<223> Description of Artificial Sequence:caveolin
      binding motif

<400> 72
Trp Xaa Phe Xaa Xaa Xaa Xaa Trp Xaa Trp
1               5                   10

<210> 73
<211> 10
<212> PRT
<213> Artificial Sequence

<220>
<223> Description of Artificial Sequence:caveolin
      binding motif

<400> 73
Trp Xaa Tyr Xaa Xaa Xaa Xaa Trp Xaa Trp
1               5                   10

<210> 74
<211> 10
<212> PRT
<213> Artificial Sequence

<220>
<223> Description of Artificial Sequence:caveolin
      binding motif

<400> 74
Trp Xaa Trp Xaa Xaa Xaa Xaa Trp Xaa Trp
1               5                   10

<210> 75
<211> 10
<212> PRT
<213> Artificial Sequence

<220>
<223> Description of Artificial Sequence:caveolin
      binding motif

<400> 75
Trp Xaa Phe Xaa Xaa Xaa Xaa Tyr Xaa Phe

1                   5                   10

<210> 76
<211> 10
<212> PRT
<213> Artificial Sequence

<220>
<223> Description of Artificial Sequence:caveolin
      binding motif

<400> 76
Trp Xaa Tyr Xaa Xaa Xaa Xaa Tyr Xaa Phe
  1               5                   10

<210> 77
<211> 10
<212> PRT
<213> Artificial Sequence

<220>
<223> Description of Artificial Sequence:caveolin
      binding motif

<400> 77
Trp Xaa Trp Xaa Xaa Xaa Xaa Tyr Xaa Phe
  1               5                   10

<210> 78
<211> 10
<212> PRT
<213> Artificial Sequence

<220>
<223> Description of Artificial Sequence:caveolin
      binding motif

<400> 78
Trp Xaa Phe Xaa Xaa Xaa Xaa Trp Xaa Phe
  1               5                   10

<210> 79
<211> 10
<212> PRT
<213> Artificial Sequence

<220>
<223> Description of Artificial Sequence:caveolin
      binding motif

<400> 79
Trp Xaa Tyr Xaa Xaa Xaa Xaa Trp Xaa Phe
  1               5                   10

<210> 80
<211> 10
<212> PRT
<213> Artificial Sequence

<220>
<223> Description of Artificial Sequence:caveolin
      binding motif

<400> 80
Trp Xaa Trp Xaa Xaa Xaa Xaa Trp Xaa Phe
  1               5                   10

<210> 81
<211> 10
<212> PRT
<213> Artificial Sequence

<220>
<223> Description of Artificial Sequence:caveolin
      binding motif

<400> 81
Trp Xaa Tyr Xaa Xaa Xaa Xaa Phe Xaa Phe
  1               5                   10


<210> 82
<211> 10
<212> PRT
<213> Artificial Sequence

<220>
<223> Description of Artificial Sequence:caveolin
      binding motif

<400> 82
Trp Xaa Trp Xaa Xaa Xaa Xaa Phe Xaa Phe
  1               5                   10


<210> 83
<211> 10
<212> PRT
<213> Artificial Sequence

<220>
<223> Description of Artificial Sequence:caveolin
      binding motif

<400> 83
Trp Xaa Tyr Xaa Xaa Xaa Xaa Phe Xaa Tyr
  1               5                   10


<210> 84
<211> 10
<212> PRT
<213> Artificial Sequence

<220>
<223> Description of Artificial Sequence:caveolin
      binding motif

<400> 84
Trp Xaa Trp Xaa Xaa Xaa Xaa Phe Xaa Tyr
  1               5                   10


<210> 85
<211> 10
<212> PRT
<213> Artificial Sequence

<220>
<223> Description of Artificial Sequence:caveolin
      binding motif

<400> 85
Trp Xaa Tyr Xaa Xaa Xaa Xaa Phe Xaa Trp
  1               5                   10

<210> 86
<211> 10
<212> PRT
<213> Artificial Sequence

<220>
<223> Description of Artificial Sequence:caveolin
      binding motif

<400> 86
Trp Xaa Trp Xaa Xaa Xaa Xaa Phe Xaa Trp
 1               5                   10

## Description

### Field of the invention

The present invention is related to a compound or a pharmaceutical composition thereof for use as a medicament for the modulation of angiogenesis for the prevention and/or the treatment of various diseases and pathologies of mammals, including of human, such as ischemic heart and peripheral vascular including cerebral diseases and tumour development and for wound healing.

The present invention is also related to a method of study, testing and/or screening of new compounds or compositions which may be used for the treatment or the prevention of said various diseases and pathologies.

### Technological background of the invention

A therapeutic angiogenesis favour the development of collateral vessels to revascularise ischemic territories. Administration of angiogenic cytokines was recently proposed as an alternative to surgery or percutaneous transluminal coronary angioplasty (PTCA) for patients suffering from ischemic cardiomyopathy. This approach is hampered by two major limitations:
- the presence of a diseased and dysfunctional endothelium in ischemic tissue alters its sensitivity to angiogenic cytokines and renders their use difficult to standardise; many contradictory reports on angiogenic properties of these cytokines stemmed from inconsistencies of their effects in vitro versus in vivo, or according to the dose used;
- the need to maintain a high local concentration of cytokines is a special challenge given numerous side effects, i.e. hypotension, consecutive to the administration of high bolus doses of angiogenic factors; alternatively, the repetitive administration of recombinant proteins is too costly.

It is also known that the decrease and even more the blocking of blood circulation in specific parts of the human body may induce the necrosis of tissues that are no longer irrigated by specific affected blood vessels. It is known that coronary vessels supplying oxygen and nutrients are essential for the irrigation of the cardiac muscle. A coronary insufficiency (for instance, induced by atherosclerosis) can be corrected by angioplasty with dilatation of the vessel obstructed by a cholesterol deposit or by surgery by bridging the non-affected portions of the vessel. However, said techniques present several drawbacks or limitations like their costs, possible re-occlusion of the dilated vessel or side effects of said surgery.

NO is produced in endothelial cells by a Ca²⁺/calmodulin-dependent enzyme, the endothelial isoform nitric oxide synthase (eNOS). It has been recently shown that eNOS is located in plasmalemmal invaginations termed caveolae, and specifically interacts with cell-specific isoforms of caveolin, the structural protein of caveolae (Feron et al., 1996). Further experiments have revealed that this association leads to the inhibition of the enzyme activity and that a stable protein-protein interaction takes place between both proteins (Michel et al., 1997a; Feron et al., 1998a), i.e. eNOS and caveolin-1 in endothelial cells or eNOS and caveolin-3 in cardiac myocytes. Consensus sequences were identified within both proteins: the scaffolding domain of caveolin, a juxtamembrane region of 20 amino acids in the C-terminal moiety of caveolin (Michel et al., 1997b), and a putative caveolin binding motif, a peptide sequence rich in aromatic residues localised in the oxygenase domain of eNOS (Feron et al., 1998a; Garcia Cardena et al., 1997). In order for eNOS to be fully activated, caveolin must dissociate from the enzyme. The ubiquitous Ca²⁺ regulatory protein calmodulin (CaM) disrupts the heteromeric complex formed between eNOS and caveolin in a Ca²⁺-dependent fashion (Michel et al., 1997a; Feron et al., 1998b): caveolin serves as a competitive inhibitor of CaM-dependent eNOS activation (Michel et al., 1997b; Michel and Feron, 1997).

As a corollary the activity of eNOS in cells is influenced by changes in the equilibrium between these two protein-protein interactions upon stoechiometric changes in the abundance of either of these partners. It has been shown that in cardiac myocytes, the transfection of a caveolin-3 construct or the loading of a synthetic peptide corresponding to caveolin scaffolding domain leads to the decrease in NO production at the basal level as well as following agonist stimulation (Feron et al., 1998c). Furthermore, exposure to high cholesterol concentrations decreases NO production through an upregulation of the abundance of endogenous caveolin-1 (mediated by a sterol regulatory element in its promoter region) and the subsequent inhibitory heterocomplex formation with eNOS (Feron et al., 1999). Functional consequences following changes in the abundance of caveolin-3 or the use of caveolin-like peptides have been illustrated in models of cultured myocytes in vitro, with alterations of the sensitivity of their beating rate to parasympathetic stimulation (Feron et al., 1998c)

Due to its complex biochemistry in biological milieu, NO release from exogenous donor drugs does not recapitulate all the effects of NO as produced endogenously by nitric oxide synthases. Therefore, it is necessary to develop specific biochemical processes that are involved in the promotion or the inhibition of nitric oxide release, which finds applications in the prevention and/or the treatment of various diseases and pathologies like ischemic heart and peripheral vascular including cerebral diseases, wound healing and/or tumour development.

However, today, there is no published demonstration of the impact of the alterations in the abundance of caveolin-1 on the physiological behaviour of endothelial cells (downstream from their production of NO).

3-Hydroxy-3-methylglutaryl coenzyme A (HMGCo A) reductase inhibitors (or statins) were shown to substantially reduce cardiovascular morbidity and mortality in clinical primary and secondary prevention trials (Maron et al. 2000). Although it was reasonable to attribute most (if not all) of these therapeutic benefits to the reduction in atherogenesis secondary to their effect on serum lipid profile, recent studies suggested otherwise. Indeed, statins reduced clinical end points before any measurable regression in atherosclerotic plaques (MAAS. The Multicentre Anti-Atheroma Study, 1994), diminished cardiovascular mortality even in patients with average cholesterol levels (Sacks et al. 1996; Byington et al. 1995) and restored normal endothelial function independently of their effects on serum cholesterol levels (O'Driscoll et al., 1997). These clinical benefits apparently unrelated to the central (hepatic) effect of statins to reduce LDL cholesterol have been explained by several mechanisms (the so-called pleiotropic effects of statins), including prevention of intimal thickening through induction of vascular smooth muscle cell (VSMC) apoptosis (Guijarro et al, 1998) and inhibition of VSMC migration (Hidaka et al. 1992) and proliferation (Corsini et al. 1998; Rogler et al. 1995), down-regulation of monocyte chemotaxis and neutrophil-endothelial interaction (Dunzendorfer et al. 1997), increase in fibrinolytic activity (Essig et al. 1998), plaque stabilization (Williams et al. 1998) and up-regulation of eNOS expression (Hernandez-Perera et al., 1998; Laufs et al. 1998) and/or activity (Kaesemeyer et al. 1999). Although in most of these studies, the effect of statins have been ascribed to the inhibition of the mevalonate-dependent geranylgeranylation of Rho GTPase proteins, the causal relationship between this phenomenon and the protective effect of statins on vessel function remains elusive.

Following patents/applications can be found: The document JP-10087698 describes the use of human heart caveolin peptide or its corresponding DNA for preventing and treating diabetes, obesity, cancer, arteriosclerosis and muscular dystrophy. This document relates to the use of the muscle-specific isoform of caveolin (or caveolin-3) but does not refer to the use of endothelial caveolin isoform (caveolin-1). Methods for diagnosis, evaluation and treatment of hormone-resistant cancers using caveolin antisense sequences to target tumour cells are covered in WO99/22773; certain cancers may be treated by therapies which suppress expression of the caveolin-1 gene. WO99/46592 targets proliferating cells by increasing the caveolin-1 to decrease mitosis.

It is also known to obtain a modulation of the NO production by the use of L-arginine-derived drugs or various drugs mixtures (as described in the documents WO98/48826, WO95/11014, US-5,767,160 and US-5,543,430) or with strategies based upon the exploitation of eNOS sequence (as described in the documents WO98/02555, JP-10136989, WO96/01902, WO93/18156), to target atherogenesis and restenosis (as described in the document US-5,428,070).

The production of statins and semi synthetic statins are described in WO9837220, AU6726398, WO9910499 and EP0971913. Statins are described to be used for the treatment of viral diseases (FR2646353, ZA9106638), eye complains (CA2018209, EP0402203, JP3034934, US5134124), angina pectoris, atheroscerosis, combined hypertension and hyperlipidia (WO9911263), hypertension (US4749687, EP0155809, US4755592, EP0165151) vascular diseases (WO9930704, WO9930706) and cardiac risk (WO9911263, Pfizer, WO9947123).

The present invention aims to provide new compounds, compositions and/or methods which may improve the prevention and/or the treatment of various angiogenesis related diseases or pathologies of mammals, including the human, which do not present the drawbacks of the state of the art. Another aim of the present invention is to provide a method of study, testing, screening and manufacturing of new compounds or compositions which influences the angiogenesis.

The present invention is related to a compound for use as a medicament in the modulation of angiogenesis through the tackling of the intracellular free cholesterol-caveolin1-eNOS-NO pathway. With "tackling" it is meant that activities of the molecules that form part of this pathway can be promoted or inhibited and/or concentrations of these can be increased or decreased resulting in a modulated angiogenesis. Increase in angiogenesis would be beneficial in a variety of ischemic cardiovascular diseases. Inventors showed that molecules such as statins decrease caveolin-1 abundance ensuring more eNOS activity; more NO resulting in an increased angiogenesis. Decrease in angiogenesis would be beneficial in angiogenesis-dependent tumour growth and metastatic diseases through drugs that increase intracellular free cholesterol, and thereby increase caveolin-1 abundance.

The inventors showed for the first time that angiogenesis can be modulated directly through the modulation cholesterol metabolism and its effect on NO production.

Several different angiogenic activators have been described sofar. These include, but are not limited to FGF, VEGF and HGF. These growthfactors bind and activate specific receptor tyrosine kinases within the endothelial cells that are coupled to a variety of signal transduction pathways, most probably the Ras-p42/44 MAP kinase pathway. Liu et al. (1999) showed that both angiogenesis activators and inhibitors have also an effect on the expression of caveolin-1 and suggest that down-regulation of caveolin-1 by angiogenic growth factors may be important for endothelial cell proliferation and subsequent angiogenesis. Nevertheless, a direct link between caveolin-modulated NO increase and angiogenesis has never been made. Caveolin is a multifunctional protein known to influence many pathways or signal transduction cascades according to the cell type or the tissue origin. Caveolin may, for instance, interact with MAPK, Src-family tyrosine kinases, adenylate cyclase and G protein-coupled receptors (Smart et al., 1999). Consequently, it is not obvious to associate the effect of cytokines with caveolin-regulated NO production. The inventors proved for the first time that NO has a direct effect on angiogenesis through changes in the abundance of the scaffolding protein caveolin.

Statins inhibit the HMG-CoA reductase and have a beneficial effect in patients with coronary heart disease through the reduction in atherogenesis and the correction of associated endothelial dysfunction (Maron et al., 2000). Although statins have been shown to increase NO availability (Laufs et al., 1998; Hernadez-Perera et al., 1998; Kaesemeyer et al., 1999; Wagner et al., 2000), to improve vasodilatation (O'Driscoll et al., 1997) and to modulate the proliferation and the programmed cell death of vascular smooth cells (Guijarro et al., 1998; Corsini et al., 1998), it has never been linked to its modulating effect on angiogenesis. The inventors proved that modifying the intracellular cholesterol content (using statins) influence angiogenesis directly via this newly described intracellular free cholesterol-caveolin1-eNOS-NO pathway.

With the "intracellular free cholesterol-caveolin1-eNOS-NO pathway" is meant that there exists a direct link between all members of this pathway, wherein members are caveolin, eNOS, NO, HMGCoA reductase, HMGCoA synthase and the other enzymes of the mevalonate pathway (Goldstein and Brown, 1990), calmodulin, Hsp90, LDL and HDL receptors,. The present invention is directed to all the members of this pathway as pharmaceutical target to influence angiogenesis.

The experimental data shown below directly implicate the inhibition by atorvastatin of cholesterol synthesis in endothelial cells as the mechanism promoting NO release (experiment 1). In addition, inventors showed for the first time that statins have a promoting effect on tube formation by this effect on caveolin abundance (experiment 2 and 3).

By reducing the cholesterol content in endothelial cells, the inventors have discovered unexpectedly that in endothelial cells, the caveolin-1 down-regulation is correlated with an increase in NO release, probably by a stoechiometric regulation of eNOS activity by a cellular pool of caveolin. In addition, exposure to high cholesterol concentrations decreases NO production through an upregulation of the abundance of endogenous caveolin-1.

In peripheral cells, cholesterol homeostasis is mostly achieved through feed-back regulation of the expression of key proteins involved in sterol flux and metabolism, e.g. LDL receptor, HMG CoA synthase and reductase (Fielding and Fielding, 1997). In addition, the balance between external and internal cholesterol is maintained through the efflux of free cholesterol to high density lipoproteins (HDL), a process involving discrete plasmalemmal microdomains termed caveolae (Fielding and Fielding, 1997; Bist et al. 1997). Recently, the inventors demonstrated in endothelial cells that the level of expression of caveolin-1, the main structural component of caveolae, is directly related to the amount of extracellular LDL-cholesterol and subsequent cholesterol uptake by these cells (Feron et al., 1999). Importantly, the inventors documented that the increase in caveolin abundance induced by high LDL-cholesterol promotes its inhibitory interaction with eNOS, resulting in a decrease in NO production (Feron et al., 1999). This mechanism of cholesterol-induced impairment of NO production may participate in the proath-erogenic effects of hypercholesterolemia and more generally in the pathogenesis of endothelial dysfunction, i.e. the deterioration of the vasodilator function of the endothelium and the dysregulation of endothelial-blood cell interactions (which may lead to localized inflammation and thrombosis). Inventors hypothesized that by reducing circulating LDL-cholesterol, or directly inhibiting cholesterol synthesis in endothelial cells, statins could reverse endothelial dysfunction by decreasing caveolin expression and promoting NO release through the destabilization of the inhibitory caveolin/eNOS complex.

To test this hypothesis, the inventors incubated endothelial cells with increasing doses of the HMGCoA reductase inhibitor atorvastatin and studied the effects on caveolin protein expression levels, caveolin/eNOS interaction and eNOS activity. These experiments were performed in absence and in presence of human LDL-cholesterol fractions in order to verify the modulation of NOS activity by the statin in conditions of significant cholesterol influx from an extracellular source. Results show that very low doses of atorvastatin (0.01-0.1 µmol/L) significantly reduced caveolin abundance and restored basal and agonist-stimulated NOS activity by altering the stoichiometry of eNOS complexation with caveolin and Hsp90, thereby underlying a novel regulation of eNOS activity by atorvastatin at the post-translational level.

Tackling of the proposed "intracellular free cholesterol-caveolin1-eNOS-NO pathway" for the modulation of angiogenesis can be performed at different levels. A compound can have an angiogenic effect via the modulation of the cholesterol metabolism (HMGCoA reductase, HMGCoA synthase and the other enzymes of the mevalonate pathway, LDL and HDL receptors) or via targets which are located downstream within this pathway such as caveolin-1, calmodulin, Hsp90, eNOS and NO.

According to a preferred embodiment of the invention the compound for use as a medicament for the modulation of angiogenesis results in the modulation of the intracellular free cholesterol concentration (cholesterol metabolism and/or cholesterol flux). The inventors showed that high cholesterol concentrations decreases NO production through an upregulation of the abundance of endogenous caveolin-1 and the subsequent inhibitory heterocomplex formation with eNOS (Feron et al., 1999). In addition, the inventors showed that this could be reversed by the addition of a statin (experiment 1) and that modifying the intracellular cholesterol content influence angiogenesis directly (experiment 2 and 3). Consequently, angiogenesis can be stimulated through the downregulation of the caveolin-1.

The present invention is related to a compound for use as a medicament for the modulation of angiogenesis which is chosen from the group comprising HMGCoA reductase inhibitors or a pharmacologically acceptable derivative thereof. The inventors showed that said inhibitors influence cholesterol metabolism resulting in the decrease of the caveolin-1 abundance thereby increasing endothelium proliferation and resulting in the stimulation of angiogenesis.

According to present invention atorvastatin influences NO production and angiogenesis. Therefore a preferred embodiment of present invention descibes the use of atorvastatin for use as a medicament for the modulation of angiogenesis. Compounds with similar structures such as mevastatin, lovastatin, simvastatin, pravastatin, fluvastatin and cerivastatin will result in the same effect as described for atorvastatin.

Increase in caveolin-1 abundance can be achieved with drugs referred to as "ACAT inhibitors" ("inhibitors of Acyl-co-A Cholesterol Acyl Transferase). Therefore said compounds can be used as a medicament for the modulation of angiogenesis. ACAT inhibitors decrease the storage of cholesterol as cholesteryl-esters, thereby increasing intracellular cholesterol, that in turn, will increase caveolin-1 expression. ACAT inhibitors include the following: avasimibe, NTE122, compound 58-035, TS-962 and the bacterial product epicochlioquinone A. It is obvious for a person skilled in the art that a pharmacologically acceptable derivative thereof will also result in the same effect.

Preferably, said ACAT inhibitor is used as a medicament for the modulation of angiogenesis is chosen from the group comprising avasimibe, NTE122, compound 58-035, TS-962 and the bacterial product epicochlioquinone A.

The present invention also relates to a compound for use as a medicament for the modulation of angiogenesis and able to increase the export of cholesterol out of peripheral cells through the increased abundance of HDL particles. The increased export of cholesterol decreases the intracellular cholesterol content resulting in the decrease of the caveolin-1 expression.

Preferably, a compound for use as a medicament for the modulation of angiogenesis which is chosen from a group comprising fenofibrate, bezafibrate and ciprofibrate. It is obvious for a person skilled in the art that a pharmacologically acceptable derivative thereof will also result in the same effect.

The present invention also relates to a compound for use as a medicament for the modulation of angiogenesis which decreases the production of cholesterol-rich VLDL particles by the liver.

Preferably, a compound for use as a medicament for the modulation of angiogenesis is chosen from a group comprising nicotinic acid. It is obvious for a person skilled in the art that a pharmacologically acceptable derivative thereof will also result in the same effect.

The present invention also relates to a compound or composition for use as a medicament for the modulation of angiogenesis influencing abundance and/or activity of caveolin, eNOS, calmodulin or Hsp90. Abundance can be modulated by the addition of active recombinant molecules (peptide or corresponding coding oligonucleotides) or influencing their endogenous production.

A preferred embodiment of the invention relates to a compound or composition for use as a medicament for the modulation of angiogenesis comprising recombinant caveolin-1, recombinant eNOS, recombinant calmodulin, recombinant Hsp90 or a pharmacologically acceptable derivative thereof. In this approach the concentration of active molecules is modulated. With a "pharmacologically acceptable derivative" is meant a functional part of the polypeptide or even a chemical molecule mimicking the structural properties thereof. These molecules can be produced externally and added to the cell or produced intracellularly by adding expression vectors carrying respective coding regions and expression signals allowing the production of active polypeptides. Therefore, the present invention also relates to a compound for use as a medicament for the modulation of angiogenesis which is a nucleic acid encoding the partial or total amino acid sequence of caveolin-1 or an analogue which can increase the caveolin-1 concentration in the cell thereby increasing the scavenging of the endogenous eNOS and reducing angiogenesis. Inhibition of angiogenesis is especially desired in the treatment of solid tumours and their metastases thereby preventing nutrient and oxygen supply of the cancer cells. Present invention shows that angiogenesis is directly linked to the increased production of NO and consequently linked to the decrease of endogenous caveolin-1. Inventors therefore suggest the use of caveolin-1 sense sequences for the treatment of cancer; this is the opposite of WO99/22773 where the use of caveolin-1 antisense molecules is suggested to target hormone-resistant cancers. Alternatively, the present invention describes a compound for use as a medicament for the modulation of angiogenesis which is a nucleic acid encoding the partial or total amino acid sequence of eNOS or an analogue thereof which can increase the eNOS concentration and/or activity in the cell thereby increasing the production of NO. Which expression signals are needed for the efficient expression of respective proteins is known by a person skilled in the art. Expression can be constitutive or controlled in a specific manner.

The present invention also relates to a compound for use as a medicament for the modulation of angiogenesis which is able to change the concentration of endogenous caveolin-1, eNOS, calmodulin or Hsp90 resulting in the modulation of angiogenesis. Said change can result from the modulation of the expression of respective molecules thereby interfering with promotor/silencer/activator molecules influencing the messenger RNA population or by influencing the translation or stability of respective messenger RNA.

According to a preferred embodiment of present invention the compound for use as a medicament for the modulation of angiogenesis may be an antisense nucleic acid sequence able to hybridise with a corresponding nucleotide sequence encoding the caveolin-1 thereby antagonising the expression of the caveolin-1 protein in the cell.

Said antisense nucleic acid is able to hybridise with a corresponding sequence encoding the partial or total amino acid sequence of caveolin-1, preferably comprised into a recombinant expression vector, under the control of a regulatory sequence that allows its expression (preferably its high expression) in a transfected cell. Preferably, SEQ ID NO 5 is used as antisense sequence. The vectors that can be used for the integration of said genetic sequence are plasmids or viral vectors (such as adenoviruses), which are able to be used for the transfection of endothelial cells in vitro, in vivo or ex-vivo.

A preferred embodiment of the invention relates to a compound for use as a medicament for the modulation of angiogenesis consisting of an antagonist or agonist of caveolin-1, eNOS, calmodulin or Hsp90. Said antagonists can antagonise the function of the molecule per se or act as a competitive inhibitor by inhibiting the binding of binding partners. A competitive inhibitor can also be defined as being a scavenging molecule or a molecule able to trap one of the molecules defined to take part in the intracellular free cholesterol-caveolin1-eNOS-NO pathway.

Another preferred embodiment of the invention relates to a compound for use as a medicament for the modulation of angiogenesis which is able to trap the endogenous caveolin-1 preventing its binding to the endothelial isoform nitric oxide synthase (eNOS). Possible compounds according to the invention are short peptidic sequences corresponding to the active sites upon eNOS to caveolin-1 such as described below. Said peptides can be made synthetically or produced in a cellular system. Systems that can be used to produce such proteins are known by the person skilled in the art.

According to present invention the compound for use as a medicament for the modulation of angiogenesis may also be a nuceic acid preferably comprised into a vector encoding the partial or total amino acid sequence of eNOS as described above or the eNOS sequence mutated or deleted in the active (caveolin) binding site or an analogue thereof which can increase the concentration of unbound (activated) eNOS. Possible compounds according to the invention are short peptidic sequences corresponding to the active sites (having preferably at least the common pattern SEQ ID NO 4, more preferably the pattern SEQ ID NO 6 to 86) upon eNOS to the caveolin-1 scaffolding domains A and B (described in the following sequences SEQ ID NO 2 and SEQ ID NO 3).

The present invention also relates to a compound for use as a medicament for the modulation of angiogenesis which is able to trap the endogenous eNOS mimicking the caveolin-1 molecule, blocking its active site and preventing the NO synthesis. Possible compounds according to the invention are short peptidic sequences corresponding to the active sites upon caveolin-1 to the eNOS such as described below.

According to present invention the compound for use as a medicament for the modulation of angiogenesis may be a nucleotide sequence encoding the partial or total amino acid sequence of caveolin-1. Alternatively, an analogue to these can be used. Increase in caveolin-1 concentration in the cell results in the increase scavenging of the endogenous eNOS. Scavenging eNOS decreases the production of NO thereby carrying an inhibiting effect on angiogenesis.

In a preferred embodiment of the invention said partial amino acid sequence comprises the caveolin-1 scaffolding domains A and/or B (described in the following sequences SEQ ID NO 2 and SEQ ID NO 3) or portions thereof able to bind selectively upon the endothelial isoform of nitric oxide synthase (eNOS). The inventors reported previously that the coimmunoprecipitation of eNOS with caveolin is completely and specifically blocked by an oligopeptide corresponding to the caveolin scaffolding domain (Michel et al. 1997). Peptides corresponding to this domain markedly inhibit NO synthase activity. This inhibition is competitive and completely reversible by Ca²⁺-calmodulin. The use of these peptides have been suggested to regulate eNOS activity but never linked to a possible effect on angiogenesis. As caveolin-1 is a peptide (nucleotide sequence enclosed see SEQ ID NO 1), said analogs or compounds can be derivatives of said peptide, peptidomimetics or homologous peptides that comprise the deletion or the replacement of one or more amino acids in the original caveolin-1 sequence, or antibodies directed against the ligand binding site epitopes of the active site(s) upon the endothelial isoform of nitric oxide synthase, or anti-idiotypic antibodies directed against particular antibodies directed against the specific portions of caveolin-1 (scaffolding domain A and/or B of caveolin-1 (SEQ ID NO 2 and SEQ ID NO 3) binding the active site(s) (of caveolin-1) upon the endothelial isoform of nitric oxide synthase. Said antibodies can be raised to caveolin-1 fragments and analogs both in the unnatural occurring form or in the unrecombined form.

The present invention also relates to a pharmacological composition for use as a medicament for the modulation of angiogenesis comprising a compound or a pharmacologically acceptable derivative thereof. The composition according to the invention may comprise one or more active compounds having possible synergistic effects and a suitable pharmaceutical carrier or adjuvant that may vary according to the mode of administration.

Said suitable pharmaceutical carrier or adjuvant is a common pharmaceutical carrier or adjuvant well known by the person skilled in the art and used to increase or modulate the therapeutical and/or prophylactic effects of the active compounds according to the invention and/or to decrease the possible side effects of said compound(s).

The pharmaceutical composition according to the invention is prepared according to the methods generally applied by pharmacists and may include solid or liquid non-toxic and pharmaceutically acceptable carrier(s) or vehicle(s).

The percentage of active product / pharmaceutically suitable carrier can vary within very large ranges, only limited by the tolerance and the level of the habit forming effects of the composition to the mammal (including the human). These limits are particularly determined by the frequency of administration.

The present invention relates to the use of a compound optionally combined with a suitable excipient, for the treatment of angiogenesis related diseases such as angiogenesis-dependent tumour growth and metastatic diseases, ischemic heart and peripheral vascular diseases including cerebral diseases and wound healing. The decrease or the increase of nitric oxide results in a modification of neo-angiogenesis which has prophylactic or therapeutic properties in specific pathologies and diseases of mammals (including the human), and preferably selected from the group consisting of: high blood pressure, cardiac insufficiency, cardiac decompensation, ischemic cardiomyopathy, dilated or post-transplantation cardiomyopathies, angina pectoris (including instable angina), coronary spasm, post-transplantation coronaropathy, hypercholesterolemia, hyperlipidemia, hypertriglyceridemia, vascular side effects of diabetes melitus (insulino-dependent or not), vascular side effects of chronic renal insufficiency (uremia), endothelial dysfunction of various origins (atherosclerosis, smoke-addiction, syndrome X, obesity, hypertension, dyslipidemia, resistance to insulin), systemic or auto-immune vasculitis, hyperhomocysteinemia, buerger angeitis, post-angioplasty coronary restenosis, peripheral vascular disease, thrombo-embolic disease, deep or superficial vein thrombosis, atherosclerosis with arterial insufficiency in a vascular area (ischemia, including celebral ischemia, coronary ischemia, ...), pulmonary arterial hypertension, side effects of hemodialysis or peritoneal dialysis, various neoplastic diseases (including carcinogenesis, tumoural development and metastases proliferation), resistance of malignant neoplastic tumours to radio or chemotherapy, bladder cancer metastatic or not (cystadenocarcinoma), angiosarcoma, proliferative retinopathies, wound healing or a mixing thereof. For cancer treatment inhibition of this angiogenesis is required and therefore increase of caveolin-1 is pursued. Contrary to the present invention WO99/46592 targets proliferating cells by transfecting caveolin-1 cDNA to increase cholesrol efflux and consecutively decrease mitosis. The present invention is focused by the fact that it promotes the increase in caveolin-1 in endothelial cells (for instance through an increase in cell cholesterol) to reduce endothelial NO production and tumour angiogenesis.

In particular, the present invention also provides a diagnostic kit for the testing of a compound or a composition for their ability to modulate angiogenesis via the intracellular free cholesterol-caveolin1-eNOS-NO pathway.

The present invention describes a method for screening compounds or compositions which modulate angiogenesis via the intracellular free cholesterol-caveolin1-eNOS-NO pathway.

The present invention relates also to a method to manufacture a medicament for the modulation of angiogenesis comprising as described above.

In addition, the present invention illustrates a method of treating a subject in the need of influencing angiogenesis by administering an angiogenesis-modulating-compound according to claims 1 to 23 in a sufficient concentration able to modulate angiogenesis within this subject.

The present invention also relates to the use of a compound for the modulation of the cholesterol synthesis, influx, efflux and/or metabolism, in a cell in vitro, in vivo or ex vivo.

The present invention also relates to the use of a compound for the modulation of the expression/activity of caveolin-1 in a cell in vitro, in vivo or ex vivo.

The present invention also relates to the use of a compound for the modulation of the expression/activity of eNOS in a cell in vitro, in vivo or ex vivo.

The present invention also relates to the use of a compound for the modulation of the expression/activity of calmodulin in a cell in vitro, in vivo or ex vivo.

The present invention also relates to the use of a compound for the modulation of the expression/activity of Hsp90 in a cell in vitro, in vivo or ex vivo.

The following examples and figures merely serve to illustrate the invention and are by no way to be understood as limiting the present invention

### Brief description of the figures

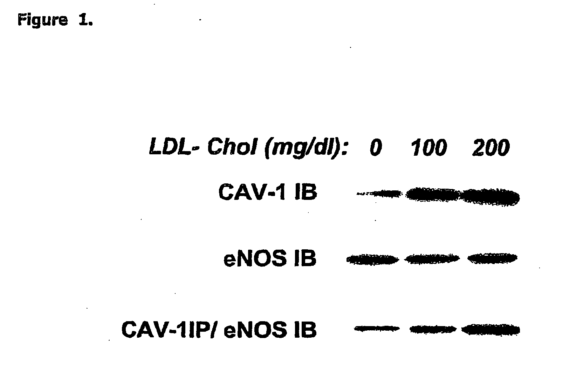

**Figure 1.** Effect of LDL-Chol on caveolin-1, eNOS proteins expression and their interaction in EC. Changes in caveolin-1 (upper lane) and eNOS (middle lane) abundance analysed by immunoblotting (IB), and in the amount of eNOS co-immunoprecipited (IP) with caveolin (lower lane) are shown. Blots are representative of 3-5 separate experiments.

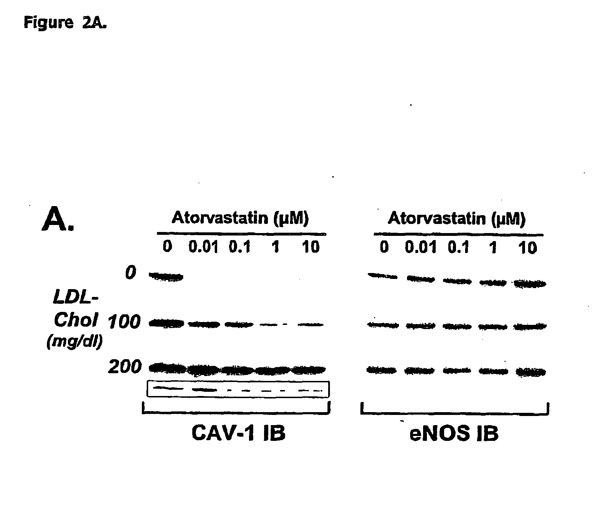

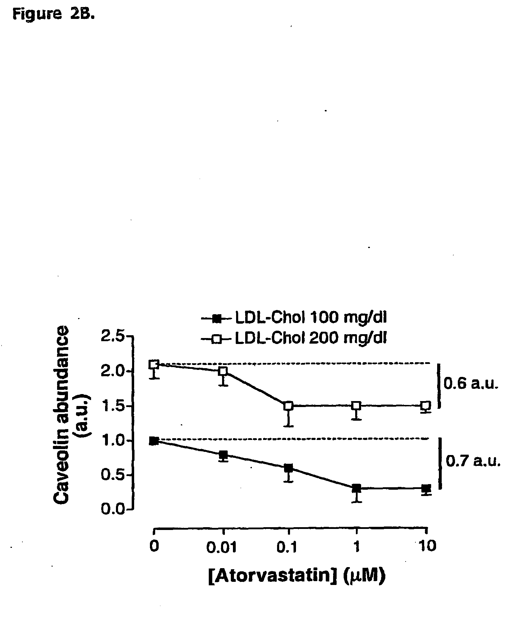

**Figure 2.** Concentration-dependent effect of atorvastatin on caveolin-1 and eNOS expression at various levels of LDL-Chol (0, 100 and 200 mg/dl). **A.** Immunoblotting (IB) analyses of caveolin-1 (left panel) and eNOS (right panel) abundance are shown. **B.** Densitometric analyses of caveolin immunoblots (n=3-4) as illustrated In panel A.

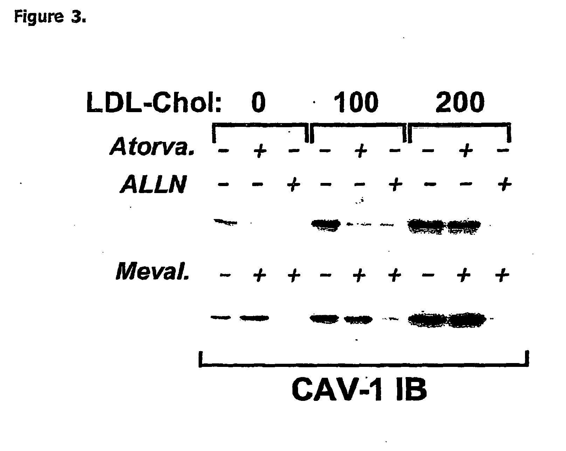

**Figure 3.** Differential sensitivity of caveolin-1 expression to cholesterol uptake and synthesis. Upper panel: Comparison of the effects of atorvastatin (1 µmol/L) or ALLN (25 µmol/L), an inhibitor of SREBP catabolism, on caveolin abundance in EC incubated in absence or in presence of LDL-Chol. Lower panel: Reversal by mevalonate of the reduction in caveolin abundance induced by atorvastatin, but not ALLN. Caveolin immunoblots (cav-1 IB) are representative of 2-3 separate experiments.

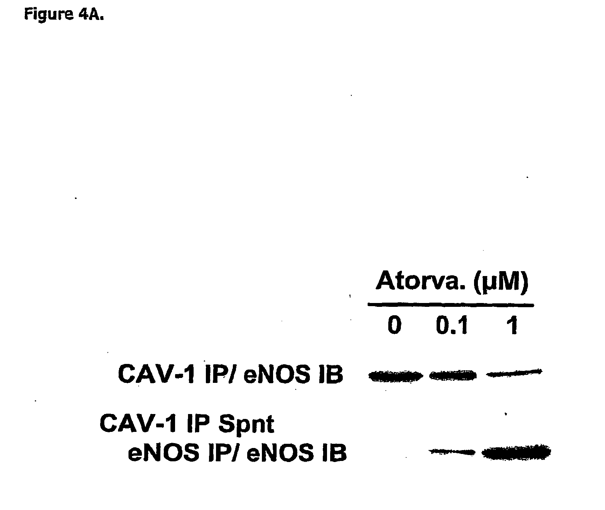

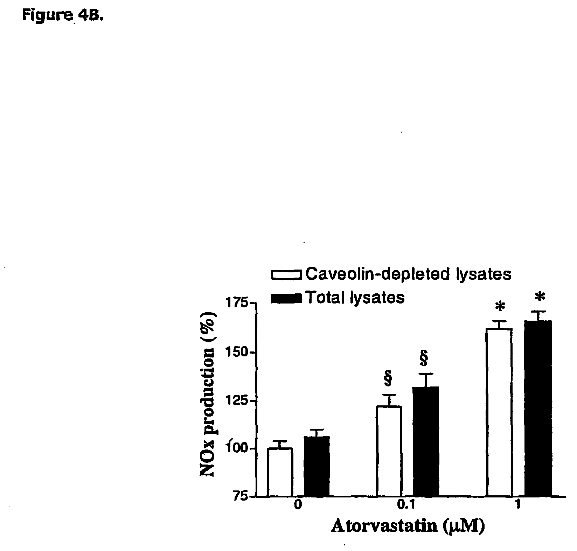

**Figure 4.** Atorvastatin decreases the inhibitory interaction of eNOS with caveolin and enhances NOS activity. EC were incubated in presence of 100 mg/dl LDL-Chol with or without atorvastatin. **A.** Effects of atorvastatin on the amount of eNOS co-immunoprecipited with caveolin (cav-1 IP, upper lane); residual eNOS immunoprecipitated by eNOS antibodies (eNOS IP, lower lane) was also measured in the caveolin IP supernatant (Spnt). eNOS immunoblots (eNOS IB) are representative of 3 separate experiments. **B.** Measurements of NOx production in caveolin-depleted and total lysates; data are expressed in % of L-NAME-inhibitable NOx production determined (in native conditions) in absence of atorvastatin, and represent the mean ± SEM of 3 separate experiments (^{§}P<0.05, *P<0.01, vs corresponding values in absence of atorvastatin treatment).

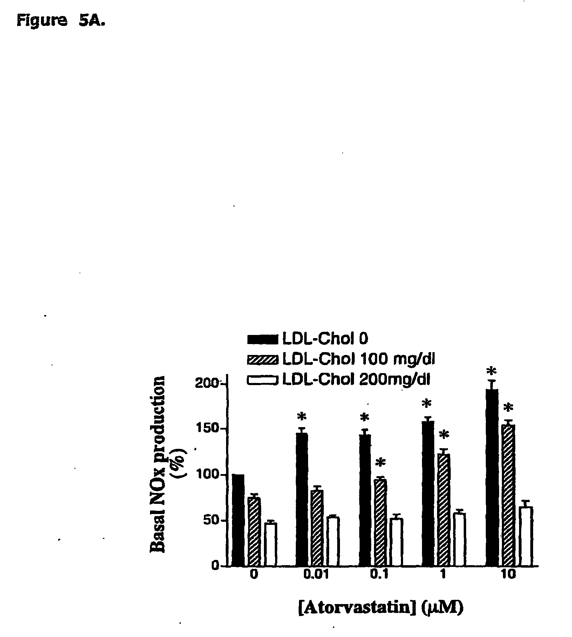

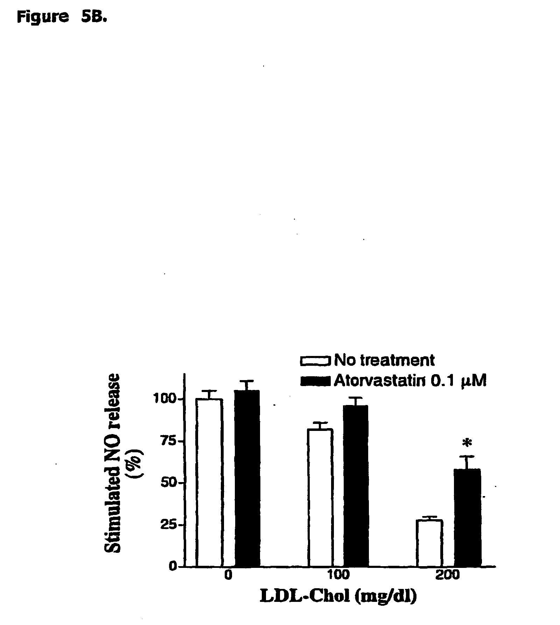

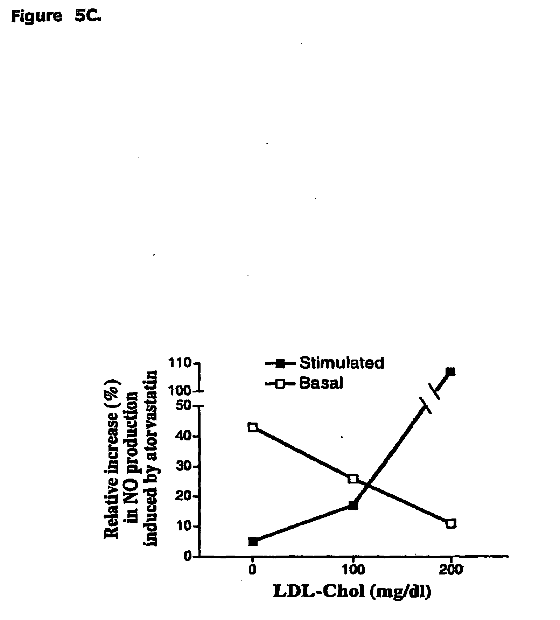

**Figure 5.** Atorvastatin differentially increased basal and agonist-stimulated NO production in intact EC. **A.** Measurements of basal NOx production from intact EC exposed to increasing concentrations of atorvastatin and/or LDL-Chol. **B.** Effects of atorvastatin (0.1 µmol/L) on A23187-induced acute NO release (over 5 min-period, measured with an amperometric probe). Data are expressed in % of L-NAME-inhibitable NOx production (panel A) or NO release (panel B) determined in absence of LDL-Chol and atorvastatin; means ± SEM are representative of 3 separate experiments. C. Schematic representation of the relationship between extracellular LDL-chol and the restoration of NO production by atorvastatin (0.1 µmol/L) (relative to untreated cells) for basal versus stimulated eNOS activity. *P<0.01, vs corresponding values in absence of atorvastatin treatment.

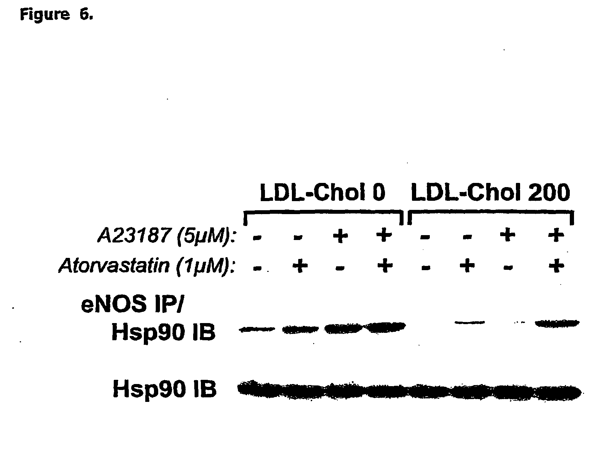

**Figure 6.** Atorvastatin differentially promotes the eNOS/Hsp90 interaction in EC incubated in absence or in presence of 200 mg/dl LDL-Chol. Immunoprecipitations were performed with eNOS antibodies (eNOS IP) from lysates of non-stimulated cells or at 30 min following an initial 5-min stimulation with A23187. Immunoprecipitates (upper lane) and lysates (lower lane) were immunoblotted with Hsp90 antibodies (Hsp90 IB). Blots are representative of 2 separate experiments.

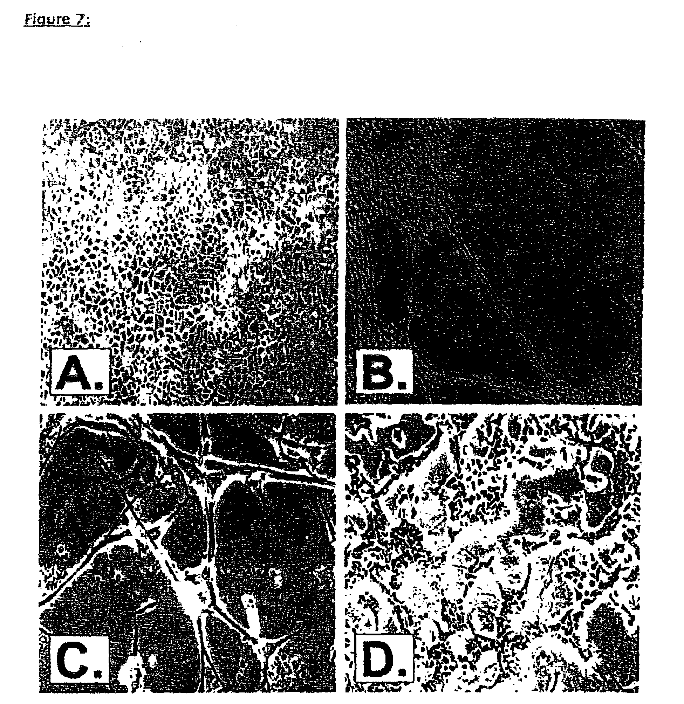

**Figure 7: A.** Pavimentous organization of endothelial cells cultured on dishes. **B.** Tube formation in the « 3D » Matrigel model. **C.** Tube formation in the « sandwich » Matrigel model. **D.** Inhibition of tube formation in caveolin-overexpressing endothelial cells in the « sandwich » model.

### Modes for carrying out the invention:

### Example 1:

HMGCoA reductase inhibition promotes endothelial nitric oxide synthase activation through a decrease in caveolin abundance. The inventors provided biochemichal and functional evidence that atorvastatin promotes NO production by decreasing caveolin-1 expression in EC, regardless of the level of extracellular LDL-Cholesterol. These findings highlight the therapeutic potential of inhibiting cholesterol synthesis in peripheral cells to correct NO-dependent endothelial dysfunction associated with hypercholesterolemia and possibly, other diseases.

### METHODS.

### Cell culture and treatments.

Human low-density lipoprotein (LDL) subfractions and lipoprotein-deprived serum (LPDS) were prepared as previously described (Feron et al. 1999). Freshly prepared LDL subfractions were supplemented with 50 µmol/L diethylenetriaminepentaacetic acid (DTPA) and used to prepare stock media at final concentrations of 100 and 200 mg/dl cholesterol.

Bovine aortic EC (BAEC) were cultured to confluence in 3.5 cm dishes in DMEM containing 10% serum and were serum-starved for 24 hours. Cell monolayers were then exposed for 48 hours to atorvastatin (10 nmol/L-10 µmol/L) in DMEM containing (or not) LDL subfraction. Incubations were carried out in presence of 100 µg/mL Cu/Zn superoxide dismutase (SOD) and medium was replaced every 12 hours. In some experiments, incubations were carried out in the presence of 1mmol/L mevalonate (Sigma) or 25 µmol/L N-acetyl-leu-leu-norleucinal (ALLN) (Boehringer Mannheim).

### Immunoprecipitation and immunoblotting.

EC were collected and homogenized in an octylglucoside-containing buffer, and processed for immunoblotting or immunoprecipitation as described previously (Feron et al. 1999; Feron et al. 1998b) . For eNOS/hsp90 co-immunoprecipitation experiments, instead, cells were homogenized in presence of 0.4% Triton X-100 and 20 mmol/L sodium molybdate as reported by Bender et al. (1999).

### NOx measurements and NO detection.

Quantitative analysis of nitrate and nitrite (NOx) was used as an index of NO production in the different cell systems. Briefly, aliquots of the medium bathing intact EC or cell lysates were collected at different time intervals and processed through a cadmium-based microreductor chamber (WPI, Aston, U.K.) to quantitatively reduce nitrate to nitrite. Acidic iodide was then used to convert nitrite to NO that was electrochemically measured with an NO-selective microsensor (WPI), as recommended by the manufacturer. In some experiments, agonist-stimulated NO release was directly monitored by the NO sensor positioned above intact cell monolayers, as previously described (Feron et al., 1999). All the experiments were carried out in presence of 7.5 U/mL SOD and adequate controls using either vehicle or NOS inhibitors were routinely performed in parallel. Data are normalized for the amount of protein in the dish or in the lysate, and are presented for convenience as mean ± SEM. Statistical analyses were made using Student's t test or one-way ANOVA where appropriate.

### RESULTS.

### LDL-Cholesterol upregulates caveolin and its interaction with eNOS in quiescent EC.

By exposing confluent, serum-starved EC for 48 hours to culture medium containing or not LDL subfractions isolated from human serum (100 or 200 mg/dl cholesterol content), the present inventors examined the extent of the modulatory effect of LDL-Cholesterol (LDL-Chol) on caveolin abundance and caveolin/eNOS interaction. As in the previous study using non-serum-starved EC (Feron et al. 1999), the inventors found that while eNOS expression was not altered by the different treatments, caveolin protein expression dose-dependently increased with the levels of LDL-Chol present in the culture medium (Figure 1). In parallel to the increase in caveolin abundance, the association between both proteins, as reflected by the fraction of eNOS immunoprecipitated by caveolin antibodies, augmented proportionally to the extracellular LDL-Chol levels (Figure 1, lower lane).

### HMGCoA reductase inhibition leads to a reduction in caveolin expression

The inventors next examined the effects of a reduction in intracellular cholesterol neo-synthesis on the same parameters. Cells were incubated for 48 hours in absence of extracellular LDL-Chol but with increasing doses of the HMGCoA reductase inhibitor atorvastatin. As depicted in Figure 2A (left panel, upper lane), the present inventors observed a dramatic reduction in caveolin expression already with the lowest dose used in this study (-75 ± 13 % with 0.01 µmol/L atorvastatin; P < 0.01, n = 3).

In order to examine whether the effect of atorvastatin on caveolin expression was maintained in the presence of an extracellular source of cholesterol, the inventors repeated the above experiments with EC exposed to either 100 mg/dl or 200 mg/dl LDL-Chol. In çells exposed to 100 mg/dl LDL-chol, the inventors observed a dose-dependent inhibitory effect of atorvastatin on caveolin expression (Figure 2A, left panel, middle lane). At 200 mg/dl LDL-Chol (Figure 2A, left panel, lower lane), despite the higher starting level of caveolin expression, a reduction in caveolin abundance was clearly detectable (see frame in Figure 2A, left panel). Furthermore, when densitometric analyses of immunoblots were performed on exposure-matched films, the absolute decrease in caveolin abundance was not significantly different in each LDL-Chol condition tested (Figure 2B). Importantly, atorvastatin (up to 1µmol/L) did not induce any significant increase in eNOS abundance, which was only observed at the highest dose of the drug (10 µmol/L) (Figure 2A, right panel).

### Caveolin expression is regulated by endogenous cholesterol synthesis and the transcriptional factor SREBP.

To examine whether these effects of atorvastatin were directly mediated through inhibition of cholesterol neo-synthesis which could transcriptionally regulate caveolin expression through sterol regulatory elements (SRE) present in its promoter region (Bist et al. 1997), two complementary sets of experiments were designed. First, EC incubated in absence or in presence of LDL-Chol were exposed to 1 µmol/L atorvastatin or 25 µmol/L ALLN, an inhibitor of SREBP catabolism, and the extent of inhibition of caveolin expression was compared. In cells incubated in absence of extracellular cholesterol, atorvastatin and ALLN inhibited caveolin expression to a similar level (Figure 3, upper panel). By contrast, when cells were exposed to 200 mg/dl LDL-Chol, atorvastatin did reduce caveolin expression but to a lesser extent than ALLN whereas at 100 mg/dl LDL-Chol, the effect of atorvastatin was intermediary (see Figure 3, upper panel). In subsequent series of experiments, the present inventors examined if mevalonate, the downstream product of HMGCoA reductase, reversed these effects. As shown in Figure 3 (lower panel), while mevalonate had no effect on the repression of caveolin expression by ALLN, it completely reversed the Inhibitory effect of atorvastatin on the expression level of caveolin in every condition tested, and even led to an increase over basal amounts of caveolin in some experiments (compare for instance lanes 1 and 2 or 7 and 8 in Figure 3, lower panel).

### HMGCoA reductase inhibition leads to a reduction in the inhibitory caveolin/eNOS interaction in EC and promotes NO production.

The inventors previously demonstrated, in the same model (Feron et al, 1999), that the extent of caveolin/eNOS interaction is proportional to the abundance of caveolin (see also Figure 1). Therefore, the inventors next verified whether the effect of atorvastatin on caveolin expression was associated with a reduction in its association to eNOS, in absence of any detectable change in eNOS abundance. Figure 4A (upper panel) shows that, upon co-incubation with 0, 0.1 or 1 µmol/L atorvastatin and 100 mg/dl LDL-Chol, atorvastatin reduced the amounts of eNOS bound to caveolin in EC, as reflected by the extent of eNOS co-immunoprecipitated by caveolin antibodies. Accordingly, more free, unbound eNOS found was found in the supernatant of the co-immunoprecipitation (Figure 4A, lower panel).

The present inventors also examined whether these changes in the extent of caveolin/eNOS interaction directly accounted for changes in eNOS activity in the same conditions. Therefore, the inventors measured eNOS activity from total lysates and caveolin IP supernatants, i.e. in caveolin-depleted lysates. As shown in Figure 4B, atorvastatin treatment led to a significant increase in NOx production in the same proportion in supernatants and total lysates, consistent with the hypothesis that in this cell model, all of the enzymatic activity is supported by the fraction of caveolin-free eNOS.

### HMGCoA reductase inhibition increases both basal and stimulated eNOS activity in intact cells.

The inventors next determined whether the decrease in caveolin abundance following statin treatment was paralleled with increases in basal and stimulated eNOS activity in intact EC. Basal eNOS activity measured from cells exposed to LDL-free medium was 0.98 ± 0.12 nmol/h/10⁶ cells (n=6). In this condition, co-incubation with increasing doses of atorvastatin produced a 45-60%-increase at concentrations between 0.01-1 µmol/L and a further 35%-increase at the highest drug concentration (10 µmol/L) (Figure 5A, black bars). Of note, when cells were co-incubated with mevalonate, statin exposure failed to induce any increase in basal NOx production (not shown). In absence of drug treatment, the 48h-incubation in presence of 100 or 200 mg/dl LDL-Chol led to an average 25 and 53 % decrease in basal NOx production (Figure 5A). When cells were co-incubated with atorvastatin, the present inventors observed a restoration of basal NOx production in cells exposed to the lower dose of LDL-Chol (100 mg/dl) but no significant increase in basal eNOS activity in cells exposed to 200 mg/dl LDL-Chol (Figure 5A).

The present inventors next examined the effect of atorvastatin on agonist-evoked eNOS activation. EC were pre-incubated or not with LDL-Chol and/or atorvastatin, and then exposed for 5 min to the calcium ionophore A23187 (5µmol/L), a receptor-independent agonist known to promote the binding of Ca²⁺-activated calmodulin to eNOS (Feron et al. 1999; Feron et al. 1998b). In absence of atorvastatin (Figure 5B, open bars), cell exposure to extracellular LDL-Chol led to a dramatic decrease in A23187-stimulated NO release consistent with the higher levels of caveolin and its inhibitory interaction with eNDS. Atorvastatin treatment (0.1 µmol/L) increased the level of agonist-induced NO release but the relative extent of this augmentation (see Figure 5B, black bars) greatly differed in each condition tested, i.e. from 5% in cells not exposed to LDL-Chol up to 17 and 107% in cells co-incubated with 100 and 200 mg/dl LDL-Chol, respectively (see also Fig. 5C).

Figure 5C illustrates the inverse relationship between extracellular LDL-Chol and the restoration of NO production by atorvastatin for basal versus stimulated eNOS activity, i.e. the effect of 0.1 µmol/L atorvastatin on basal NO production was most prominent at low extracellular cholesterol whereas the drug was most effective to potentiate agonist-evoked NO release at high LDL-Chol.

### HMGCoA reductase inhibition promotes eNOS/Hsp90 interaction.

The data presented in Figure 58 prompted the inventors to examine whether long-term activation of eNOS led to similar differences. The inventors therefore measured the amounts of NOx accumulated in the extracellular medium of EC during 30 min following a 5-min exposure to A23187 (and extensive washing). At 200 mg/dl LDL-Chol, the effect of atorvastatin was even more pronounced (than upon acute NO release, see Fig. 5B) with the NOx accumulation reaching ~500% of the value observed in absence of the statin, while NOx production increased to 1.2 and 1.6-fold the control value at 0 and 100 mg/dl LDL-chol, respectively (not shown).

Hsp90 has been proposed to act as a molecular chaperone facilitating long-term activation of eNOS (Garcia-Cardena et al., 1998). The inventors therefore compared the amount of Hsp90 co-immunoprecipitated by eNOS antibodies from lysates of cells incubated or not with atorvastatin, in presence or in absence of LDL-Chol.

The present inventors first confirmed that Hsp90/eNOS interaction was promoted upon agonist stimulation, as shown by the increased amount of Hsp90 detected in the eNOS immunoprecipitate from lysates of A23187-simulated EC (see lanes 1 and 3 in Figure 6, upper panel). Of interest, atorvastatin promoted the interaction between Hsp90 and eNOS at the basal level, in EC exposed or not to high LDL-cholesterol (see Figure 6, lanes 5 and 6, and 1 and 2, respectively). More importantly, the data show that upon stimulation with calcium ionophore, the Hsp90/eNOS interaction was minimally influenced by atorvastatin in the absence of LDL-cholesterol (see Figure 6, lanes 3 and 4), but increased five-fold by atorvastatin in the presence of 200 mg/dl LDL-cholesterol in the extracellular medium (see Figure 6, lanes 7 and 8). There was no change in total Hsp90 expression in any condition (Figure 6, lower panel), suggesting that the observed changes in the amount of Hsp90 recruited with eNOS were only determined by changes in unbound, caveolin-free eNOS.

The inventors identified a cholesterol-dependent mechanism of endothelial dysfunction that involves the post-translational regulation of eNOS in the absence of changes in absolute eNOS abundance. Instead, cholesterol modulates the abundance, in EC, of caveolin-1 that acts as an inhibitor of eNOS activation. Importantly, this study shows that the HMGcoA reductase inhibitor, atorvastatin, restores eNOS activity through downregulation of caveolin-1 expression. This occurs at concentrations as low as 10-100 nmol/L, and is fully reversed by addition of excess mevalonate, confirming the drug's specific effect on the mevalonate pathway. Recent studies had highlighted the therapeutic potential of inhibiting the mevalonate pathway in peripheral cells through the reduction of downstream isoprenoid intermediates, regardless of statins'effect on cholesterol neosynthesis. By contrast, the results directly implicate the inhibition by atorvastatin of cholesterol synthesis in EC as the mechanism promoting NO release. This is consistent with the previous identification of SRE in the promoter sequence of the caveolin-1 gene (Bist et al., 1997) and the observation that ALLN, an inhibitor of SREBP catabolism, fully abrogates caveolin-1 expression in cultured EC.

Atorvastatin treatment potentiated both basal and agonist-stimulated NO production. The use of SOD and the receptor-independent calcium ionophore, A23187 ruled out indirect effects on NO oxidation or endothelial receptor coupling to eNOS, respectively. Of note, the statin was effective both in the absence and in the presence of exogenously added LDL-cholesterol, but with different efficiency in resting or stimulated cells (see Fig. 5C). This is best rationalized in terms of the reciprocal and competitive binding of either caveolin or calcium-calmodulin on eNOS to determine its activation. In unstimulated cells, i.e. at low activated calcium-calmodulin levels, eNOS activity is expected to be mainly determined by the abundance of caveolin available for its inhibitory binding to eNDS. In this condition, the effect of atorvastatin on NO production is proportional to its reduction of total caveolin. Accordingly, the inventors observed a stronger potentiation of basal NO release at zero extracellular LDL-cholesterol, i.e. when the (low) caveolin pool is maximally sensitive to inhibition of endogenous cholesterol synthesis (see Fig. 5A). The picture is reversed in agonist-stimulated cells, in which activated calcium-calmodulin will easily displace caveolin, at least at relatively low levels of the latter. At high caveolin levels (i.e. in the presence of exogenous LDL-cholesterol), however, agonist-stimulation of eNOS activity may be more critically affected by subtle changes in total caveolin abundance, such as those observed with atorvastatin at 200 mg/dl LDL-C (see Fig. 58). These small changes may be sufficient to alter the enzyme's ability to interact with other modulators as well, as demonstrated with the chaperone hsp90 in the present study, resulting in substantial increases in eNOS sensitivity. More generally, present demonstration of atorvastatin's effect to decrease caveolin-1 expression leaves additional possibilities to impact on disease processes involving the interaction of caveolin with a variety of signaling molecules, e.g. tyrosine kinases, adenylyl cyclase or G-protein coupled receptors (Smart et al. 1999).

### Clinical implications

A deficient NO-dependent vasorelaxation is central to coronary and peripheral ischemic diseases secondary to hypercholesterolemia and may result from either a decreased production of NO or an increase in NO catabolism (Wever et al. 1998). Interestingly, both processes can be restored by supplementation with L-arginine or tetrahydrobiopterin in vivo (for refs see Wever et al, 1998), suggesting that eNOS is still expressed in the dysfunctional endothelium, but somehow inactivated. In this regard, the eNOS "sensitizing" effect of atorvastatin may already operate at very early stages of endothelial dysfunction, at a time when the enzyme's activation (but not abundance) is downregulated. Present results demonstrates that this peripheral effect can occur at very low concentrations of the drug, i.e. close to those achieved therapeutically in vivo. In addition, the observation of a marked potentiation of basal NO production at zero extracellular LDL-cholesterol may extend the clinical usefulness of atorvastatin (and perhaps other statins as well) to NO-dependent endothelial dysfunctions secondary to diseases other than hypercholesterolemia, such as hypertension or heart failure.

### Example 2:

The inventors have developed different techniques to evaluate angiogenesis in vitro and used some of them to test the possibility to modulate NO-dependent angiogenesis by altering caveolin abundance.

Indeed, while endothelial cells culture leads to pavimentous organization when plated on dishes (Figure 7A), endothelial cells progressively form tubes when cultured within gels of collagen, fibrin or Matrigel®. Accordingly, in the "3-D model", endothelial cells are mixed to the matrix before gelification and tube-like structures are obtained after 48-72 hours (Figure 7B); this model, however, reduces the efficiency of transfection. In the "sandwich model", cells are first cultured to confluence on a first layer of matrix, and are then covered by a second matrix layer; cells are easily transfected in the interval preceding the addition of the upper layer, The latter technique allows the formation of endothelial tubes in 3-6 hours; the tube structures are mature 12 hours after induction (Figure 7C) and then start to regress.

When endothelial cells were first transfected with a caveolin-encoding vector (24 hours before addition of the second matrix layer), the formation of tube structures was dramatically inhibited (Figure 7D). Importantly, SNAP, a NO donor agent, was shown to correct this anti-angiogenic effect observed in caveolin-overexpressing cells. Yet, in non-transfected cells, both SNAP and the HMGCoA reductase inhibitor atorvastatin accelerated the endothelial tube formation in this model of in vitro angiogenesis. Also, in the same model, NOS inhibitors were shown to mimick the effect of caveolin overex-pression, i.e. by slowing down the tube formation. Of note, when endothelial cells were transfected with an irrelevant construct (β-galactosidase-encoding), no change in the rate and extent of tube formation was observed.

Finally, atorvastatin was shown to promote NO production (see example 1) in the same cells (bovine aortic endothelial cells) by decreasing caveolin abundance. The inventors therefore interpret the observed promoting effect of statins on tube formation by this effect on caveolin abundance.

### Example 3:

The inventors have also developed techniques to evaluate angiogenesis ex vivo and used them to test the possibility to modulate NO-dependent angiogenesis by altering caveolin abundance.

Rat/mice aorta or arteries from fresh human umbilical cords are dissected and imbedded in a fibring gel. Endothelial cells are observed to migrate from the ends of the vessels and organize into tubules after 5-7 days.

When aorta strips are embedded in fibrin gels in presence of atorvastatin, the density of neo-formed tubes was significantly higher (+53%) than in control experiments. Interestingly, this effect was completely blocked by co-incubation with NOS inhibitors. Combined with the higher levels of caveolin in aortic endothelial cells exposed to atorvastatin, the inventors interpret the positive effect of statins on the tube outgrowth by the cholesterol-caveolin-eNOS-NO pathway described above.

### Example 4:

The inventors have also developed techniques to evaluate angiogenesis in vivo and will use them to test the possibility to modulate NO-dependent angiogenesis by altering caveolin abundance.

The Chick chorioallantoic membrane (CAM) assay models the angiogenic process in the developing chick using the accessible chorioallantoic membrane with its develping network of blood vessels. When the CAM appears on day 7 after fertilization, statins can be implanted to modulate vessels growth.

In these different assays, lipophilic and more hydrophilic statins will be used at doses known to be reached therapeutically (10 nM-100 µM). Experiments will first be performed in the presence or the absence of NOS inhibitors to validate the existence of a NO-mediated angiogenic process in these models. The inventors will then examine the effects of statins on caveolin abundance by Western Blotting and consecutively, on eNOS activability by measuring the level of interaction of the enzyme with caveolin in co-immunoprecipitation experiments, as well as by determining the amounts of nitric oxide released in each condition.

### REFERENCES

Bender AT, Silverstein AM, Demady DR et al. Neuronal nitric-oxide synthase is regulated by the Hsp90-based chaperone system in vivo. J Biol Chem. 1999;274:1472-1478.
Bist A, Fielding PE, Fielding CJ. Two sterol regulatory element-like sequences mediate up-regulation of caveolin gene transcription in response to low density lipoprotein free cholesterol. Proc Natl Acad Sci U S A. 1997;94:10693-10698.
Byington RP, Jukema JW, Salonen JT et al. Reduction in cardiovascular events during pravastatin therapy. Pooled analysis of clinical events of the Pravastatin Atherosclerosis Intervention Program. Circulation. 1995;92:2419-2425.
Corsini A, Pazzucconi F, Arnaboldi L et al. Direct effects of statins on the vascular wall. J Cardiovasc Pharmacol. 1998;31:773-778.
Dunzendorfer S, Rothbucher D, Schratzberger P et al. Mevalonate-dependent inhibition of transendothelial migration and chemotaxis of human peripheral blood neutrophils by pravastatin. Circ Res. 1997;81:963-969.
Essig M, Nguyen G, Prie D et al. 3-Hydroxy-3-methylglutaryl coenzyme A reductase inhibitors increase fibrinolytic activity in rat aortic endothelial cells. Role of geranylgeranylation and Rho proteins. Circ Res. 1998;83:683-690.
Feron O., Belhassen L., Kobzik L., Smith TW., Kelly RA. & Michel T. Endothelial nitric oxide synthase targeting to caveolae: specific interactions with caveolin isoforms in cardiac myocytes and endothelial cells. J Biol Chem. 1996;271, 22810-22814.
Feron O., Michel JB., Kazu S. & Michel T. Dynamic regulation of eNOS: complementary roles of dual acylation and caveolin interactions. Biochemistry 1998a;37:193-200.
Feron O, Saldana F, Michel JB et al. The endothelial nitric-oxide synthase-caveolin regulatory cycle. J Biol Chem. 1998b;273:3125-3128.
Feron O., Dessy C., Opel DJ., Arstall MA., Kelly RA. & Michel T. Modulation of the eNOS-caveolin interactions in cardiac myocytes: implications for the autonomic regulation of heart rate. J Biol Chem. 1998c;273, 30249-30254.
Feron O, Dessy C, Moniotte S et al. Hypercholesterolemia decreases nitric oxide production by promoting the interaction of caveolin and endothelial nitric oxide synthase. J Clin Invest. 1999;103:897-905.
Fielding CJ, Fielding PE. Intracellular cholesterol transport. J Lipid Res. 1997;38:1503-1521.
Garcia-Cardena G, Martasek P, Masters BS, Skidd PM, Couet J, U S, Lisanti MP, Sessa WC. Dissecting the interaction between nitric oxide synthase (NOS) and caveolin. Functional significance of the noscaveolin binding domain in vivo. J Biol Chem. 1997;272:5437-25440.
Garcia-Cardena G, Fan R, Shah V et al. Dynamic activation of endothelial nitric oxide synthase by Hsp90. Nature. 1998;392:821-824.
Goldstein JL, Brown MS. Regulation of the mevalonate pathway. Nature. 1990;343:425-430.
Guijarro C, Blanco-Colio LM, Ortego M et al. 3-Hydroxy-3-methylglutaryl coenzyme a reductase and isoprenylation inhibitors induce apoptosis of vascular smooth muscle cells in culture. Circ Res. 1998;83:490-500.
Hernandez-Perera O, Perez-Sala D, Navarro-Antolin J et al. Effects of the 3-hydroxy-3-methylglutaryl-CoA reductase inhibitors, atorvastatin and simvastatin, on the expression of endothelin-1 and endothelial nitric oxide synthase in vascular endothelial cells. J Clin Invest. 1998;101:2711-2719.
Hidaka Y, Eda T, Yonemoto M et al. Inhibition of cultured vascular smooth muscle cell migration by simvastatin (MK-733). Atherosderosis. 1992;95:87-94.
Kaesemeyer WH, Caldwell RB, Huang J et al. Pravastatin sodium activates endothelial nitric oxide synthase independent of its cholesterol-lowering actions. J Am Coll Cardiol. 1999;33:234-241.
Laufs U, La F, V, Plutzky J et al. Upregulation of endothelial nitric oxide synthase by HMG CoA reductase inhibitors. Circulation. 1998;97:1129-1135.
Liu J, Razani B, Tang S, Terman BI, Ware JA, Usanti MP. Angiogenesis activators and inhibitors differentially regulate caveolin-1 expression and caveolae formation in vascular endothelial cells. Angiogenesis inhibitors block vascular endothelial growth factor-induced down-regulation of caveolin-1. J Biol Chem 1999;274:15781-5.
MAAS: the Multicentre Anti- Atheroma Study: Effect of simvastatin on coronary atheroma. Lancet. 1994;344:633-638.
Maron DJ, Fazio S, Linton MF. Current perspectives on statins. Circulation. 2000;101:207-213.
Michel JB., Feron O., Sacks D. & Michel T. Reciprocal regulation of endothelial nitric oxide synthase by Ca²⁺-calmodulin and caveolin. J Biol Chem. 1997a;272:15583-15586.
Michel JB., Feron O., Prabhakar P., Sase K. & Michel T. Caveolin versus calmodulin: counterbalancing allosteric regulators of endothelial nitric oxide synthase. J Biol Chem. 1997b;272:25907-25912.
Michel T. & Feron O. Nitric oxide synthases: which, where, how and why? J. Clin. Invest. 1997;100:2310-2316.
O'Driscoll G, Green D, Taylor RR. Simvastatin, an HMG-coenzyme A reductase inhibitor, improves endothelial function within 1 month. Circulation. 1997;95:1126-1131.
Rogler G, Lackner KJ, Schmitz G. Effects of fluvastatin on growth of porcine and human vascular smooth muscle cells in vitro. Am J Cardiol. 1995;76:114A-116A.
Sacks FM, Pfeffer MA, Moye LA et al. The effect of pravastatin on coronary events after myocardial infarction in patients with average cholesterol levels. Cholesterol and Recurrent Events Trial investigators, N Engl J Med. 1996;335:1001-1009.
Smart EJ, Graf GA, McNiven MA et al. Caveolins, liquid-ordered domains, and signal transduction. Mol Cell Biol. 1999;19:7289-7304.
Wagner AH, Kohler T, Ruckschloss U, Just I, Hecker M Improvement of NO-dependent vasodilatation by HMG-CoA reductase inhibitors through attenuation of endothelial superoxide anion formation. Arterioscler Thromb Vasc Biol 2000;20:61-9.
Wever RM, Luscher TF, Cosentino F et al. Atherosclerosis and the two faces of endothelial nitric oxide synthase. Circulation 1998;97:108-112.
Williams JK, Sukhova GK, Herrington DM et al. Pravastatin has cholesterol-lowering independent effects on the artery wall of atherosderotic monkeys. J Am Coll Cardiol. 1998;31:684-691.

## Claims

1. Use of a compound selected from the group consisting of an HMGCoA reductase inhibitor, recombinant eNOS, a nucleic acid encoding the partial or total amino acid sequence of eNOS, an antisense nucleic acid as defined by SEQ ID NO 5, short peptidic sequences corresponding to the active site upon eNOS to the caveolin-1 scaffolding domains A and B having the common pattern as described in SEQ ID NO 4 and SEQ ID NO 6 to SEQ ID NO 86 or a pharmacologically acceptable derivative thereof for the manufacturing of a medicament for the stimulation of angiogenesis.

2. Use according to claim 1 wherein the pharmacologically acceptable derivative thereof is a functional part of the polypeptide or a chemical molecule mimicking the structural properties thereof.

3. Use according to claim 1 or 2, for the treatment of ischemic heart and peripheral vascular diseases including cerebral diseases and wound healing, high blood pressure, cardiac insufficiency, cardiac decompensation, ischemic cardiomyopathy, dilated or post-transplantation cardiomyopathies, angina pectoris (including unstable angina), coronary spasm, vascular side effects of chronic renal insufficiency (uremia), atherosclerosis, smoke-addiction, syndrome X, obesity, hypertension, resistance to insulin, systemic or auto-immune vasculitis, hyperhomocysteinemia, buerger angeitis, peripheral vascular disease, thrombo-embolic disease, deep or superficial vein thrombosis, atherosclerosis with arterial insufficiency in a vascular area (ischemia, including celebral ischemia, coronary ischemia, ...), pulmonary arterial hypertension, side effects of hemodialysis or peritoneal dialysis.

4. Use according to any of claims 1 to 3, wherein said HMGCoA reductase inhibitor is selected from the group consisting of atorvastatin, mevastatin, lovastatin, simvastatin, pravastatin, fluvastatin or cerivastatin.

5. Use according to any of claims 1 to 4, wherein said HMGCoA reductase inhibitor is atorvastatin.

6. Use according to any of claims 1 to 5, wherein the HMGCoA reductase inhibitor concentration used is 0.01 µM to 100 µM.

7. Use of a pharmacological composition comprising a compound as defined in any of claims 1 to 4, in the manufacture of a medicament for the stimulation of angiogenesis.

8. Use according to any of the claims 1 to 7, optionally combined with a suitable excipient, for the stimulation of angiogenesis to prevent and/or treat diseases or pathologies selected from the group comprising ischemic heart and peripheral vascular diseases including cerebral diseases and wound healing, high blood pressure, cardiac insufficiency, cardiac decompensation, ischemic cardiomyopathy, dilated or post-transplantation cardiomyopathies, angina pectoris (including instable angina), coronary spasm, vascular side effects of chronic renal insufficiency (uremia), atherosclerosis, smoke-addiction, syndrome X, obesity, hypertension, resistance to insulin, systemic or auto-immune vasculitis, hyperhomocysteinemia, buerger angeitis, peripheral vascular disease, thrombo-embolic disease, deep or superficial vein thrombosis, atherosclerosis with arterial insufficiency in a vascular area (ischemia, including celebral ischemia, coronary ischemia, ...), pulmonary arterial hypertension, side effects of hemodialysis or peritoneal dialysis.

9. Use according to claim 8, whereby the angiogenesis related diseases or pathologies are prevented and/or treated in mammals, including the human.

10. Method to manufacture a medicament for the stimulation of angiogenesis comprising the use of a compound as defined in any of claims 1 to 6.

11. Method for screening compounds or compositions which stimulates angiogenesis wherein said compound is as defined in any of claims 1 to 6 comprising the step of trapping the endogenous caveolin-1 preventing its binding to the endothelial isoform nitric oxide synthase (eNOS) and stimulating angiogenesis.
